# EUROPEAN PATENT APPLICATION

(11) **EP 2 177 237 A1**
(43) Date of publication of application: **21.04.2010**
(21) Application number: 08167030.9
(22) Date of filing: 20.10.2008
(51) Int. Cl.: A61L 27/00, A61F 2/12

(54) **Core fillings for prostheses**

(71) Applicant: Hoc Age CTA, 6271 EC Gulpen (NL)
(72) Inventor: Koole, Levinus Hendrik, 6271 EC Gulpen (NL); Dumont, Ewald Anna Wilhelmus Jozef, 6221 KM Maastricht (NL)
(74) Representative: Hatzmann, Martin

(57) **Abstract**

The invention is directed to a core filling material for prostheses, such as breast implants and prostheses for aesthetic and reconstructive surgery, comprising at least one biocompatible gel material and evenly dispersed through the gel material at least one particulate radiopaque material, wherein the core filling material is viscoelastic at 37°C, having a viscosity in the range of 10 to 10⁸ cP.

## Description

The invention is directed to core fillings for soft-tissue prostheses, such as prostheses for cosmetic, plastic or reconstructive surgery, including breast implants.

Breast augmentation involves the careful surgical implantation of a synthetic prosthesis in each breast that is treated. It has become one of the most common procedures in cosmetic and plastic surgery. Several different types of breast augmentation implants are commercially available as FDA-approved medical devices. A variety of different breast prosthetic implants has been described in the patent literature. These include the following patents: (i), US patents 7,105,116 and 6,692,527 (H.T. Bellin et al., (September 12, 2006 and February 17, 2004) describing a synthetic breast implant with anatomical shape, and with a smooth surface at the front side and a textured surface at the rear side; (ii) US patent 6,916,339 (M.-C. Missana et al., (July 12, 2005) describing an implantable breast prosthesis comprising a soft pouch capable of containing a filler such as a silicone gel or a physiological serum; (iii) WO patent application 0166039 (J. Mortensen (September 6, 2004) describing a biocompatible non-resorbing breast prosthesis consisting of a fabic with multiple layers; (iv) US patent 6,881,226 (J.D. Corbitt et al. (April 19, 2005) describing a bioabsorbable breast implant; (v) US patent 6,802,861 (R.S. Hamas (October 12, 2004) describing a surgically implantable structured breast implant with two shells that enclose a lumen, wherein said lumen can accommodate a fluid; (vi) US patent 5,500,017 (P.D. Bretz et al., (November 17, 1994), describing a breast implant formed from a polymeric sac with a filling material which is an aqueous sugar solution (preferably honey) with a viscosity of at least 15 cp at 98.6 ° F.

Breast prosthesis can be divided into three different categories:
1. Silicone-filled breast implants. These implants are produced in a natural shape, and they are soft and natural to feel. These features make them very appealing to women seeking breast augmentation. Silicone-filled breast prostheses are produced in a pre-filled form, which requires a relatively large incision to insert them. A silicone implant consists of a shell (capsule) -made out of elastomer silicone rubber- filled with *silicone gel,* or *cohesive gel.* The silicone gel is a viscous liquid, whereas the cohesive gell has a rubbery consistency with shape-memory properties. This difference becomes important if rupture of the capsule should occur. Then, silicone gels may escape from the shell into the surrounding tissues, whereas cohesive gels are claimed to retain their shape and volume as they reside within the shell cavity.
2. Saline-filled breast implants. These implants consist of a capsule of silicone elastomer rubber. The capsule is implanted through a relatively small incision, and then filled with saline, through a valve. Saline-filled breast prosthese are believed to be safer than their silicone-filled counterparts, since leakage or rupture will lead to the release of salt water only.
3. Breast prostheses that differ from categories (1) and (2), since they are constructed out of different materials. Examples of such different materials are, but are not limited to: sugar solutions to fill the interior of the breast prosthesis, biodegradable materials forming the envelope structure and/or the interior portion of the breast prosthesis.

Women may desire augmentation of one or both of their breasts for various reasons, such as: to correct different size of the breasts, to correct changes of the breasts after pregnancy, lactation, or aging. Breast implants are also implanted to correct the shape of the breast after excision of malignant tissue. Furthermore, breast augmentation is frequently performed for cosmetic reasons. Breast enhancement can give a woman more proportional shape and improved self esteem.

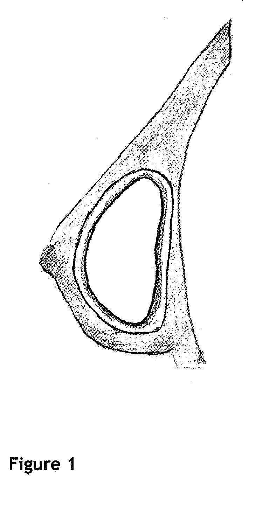

This invention provides an improved method to cope with a common problem that is associated with breast prosthetic implants, namely rupture of the capsule. This problem has been studied extensively. For example, Brown *et al.* reported a population-based study in the year 2000 [SL Brown et al., American Journal of Radiology Vol. 175, pp 1057-1064 (2000)]. The study included 344 women; the prevalence of silicone gel implant rupture was reported to be as high as 55%. In most cases, the rupture of the synthetic capsule did not lead to serious consequences. Presumably this was due to the presence of a capsule of fibrous scar tissue capsule that forms in the end-stage of the foreign-body reaction to the presence of the synthetic implant. The fibrous capsule that forms around implants usually keeps silicone gel from spreading into the surrounding breast even when the implant shell fails; see Figure 1. In these cases there is only *intracapsular leakage* of the silicone gel, which is usually unsymptomatic, although serious changes in the shape of the breast may be the result. Intracapsular leakage has also been called "uncollapsed rupture". However, when the fibrous-tissue capsule also ruptures, there is *extracapsular* spread of silicone. This will often lead to silicone granuloma formation and other unwanted reactions of tissues to the silicone gel, causing serious complaints. It has been reported in the scientific literature that extracapsular silicone may increase the risk for fibromyalgia and other connective tissue diseases [see: SL Brown et al., Journal of Rheumatology Vol. 28, pp 996-1003 (2001)]. Extracapsular leakage was found in 22 % of the ruptured implants. Other scientific publications of relevance are: A. Lahiri & R. Waters, Journal of Plastic, reconstructive and Aesthetic Surgery Vol. 59, pp 885-886 (2006) and C.M. McCarthy et al., Plastic and Reconstructive Surgery Vol. 121, pp 1127-1134 (2008).

The purpose of this invention is to improve diagnosis of breast implant rupture, i.e., to distinguish intracapsular rupture from extracapsular rupture, and to identify and localize silicone granulomas due to extracapsular leakage, or to establish integrity of a breast implant under suspicion. Several groups of researchers have described the use of magnetic resonance (MR) imaging as the technique of choice for the evaluation of breast implant integrity. Particularly axial and sagittal fast spin-echo T2-weighted images with water suppression, axial inversion-recovery T2-weighted images with water suppression, and axial T2-weighted images with silicone suppression were found to be suitable. MR imaging was claimed to reveal extracapsular silicone gel and silicone granulomas [see: DL Monticciolo, American Journal of Radiology Vol. 163, pp 5-56 (1994)]. Furthermore, sonography was reported to be particularly useful to detect extracapsular silicone [see: P Herzog, Plastic and Reconstructive Surgery Vol. 84, pp 856-857 (1980)].

A more recent study by Berg et al. indicates that rupture of breast prostheses is common, especially as implants age [see: WA Berg et al., American Journal of Radiology Vol. 178 pp 465-472 (2002)]. In their patient group, which comprised 359 women, they encountered 378 ruptured implants, and 133 of those (35 %) showed evidence of extracapsular spread of silicone. MR imaging turned out to be frequently equivocal as a method to recognize extracapsular silicone. Berg et al. found that extracapsular rupture is usually manifest as a local spread of silicone in the breast, but this is not well-depicted on fast spin-echo T2-weighted images. Berg et al. also concluded that distinction of intracapsular leakage (bulging within the fibrous capsule) from extracapsular leakage (herniation through the capsule) remains problematic. For example, confusion over contour deformity due to weakening versus breach of the capsule did arise. The main conclusion from the study of Berg et al. was that distinction of a weakened fibrous capsule from extrusion of silicone gel through the capsule remains problematic, even when advanced MR techniques are applied. In a more recent study, D.P. Gorczyca et al. reported that extracapsular rupture of existing breast prostheses can be difficult to identify, both through MR techniques and through computed tomography. It is important to differentiate normal prominent radial folds from an actual collapsed implant shell. See: D.P. Gorczyca et al., Plastic and Reconstructive Surgery Vol. 120 (Suppl. 1), pp 49S-61S (2007). Another relevant report on complications following implantation of a hydrogel breast implant was published by S.T. Adams et al. in the Journal of Plastic, Reconstructive and Aesthetic Surgery Vol. 60, pp 210-212 (2007).

It is an object of the present invention to provide for materials that can be used in prostheses (including breast implants) and which allow for an easy and reliable diagnosis method, not having the above describe disadvantages.

In a first embodiment the invention is directed to a core filling material for prostheses, such as breast implants and prostheses for aesthetic and reconstructive surgery, comprising at least one biocompatible gel material and evenly dispersed through the gel material at least one particulate radiopaque material, wherein the core filling material is viscoelastic at 37 °C, having a viscosity in the range 10 - 100.000 centiPoise (cP). The viscosity of the core filling material is determined at 37 °C with a Brookfield viscometer equipped with a #27 spindle.

According to a preferred embodiment, the viscosity of the core filling material is in the range 100 - 10.000 cP, more preferably in the range 1200-3600 cP.

The amount of radiopaque material should in general be sufficient to provide spatial information about the presence of the gel material in non-invasive X-ray imaging of the radiopaque particles. This feature will generally have been met in case the ratio of gel material: radiopaque particles (volume: volume) is in the range 100 : 1 to 1 : 100, preferably in the range 20 : 1 to 1 : 1, more preferred 9 : 1 to 3 : 1.

The radiopaque particles are preferably spheres with a diameter between 0.1 micrometer and 2 millimeter, preferably in the diameter range 50 micrometer - 1 millimeter.

The amount of radiopaque particles in the material of the invention is determined by various aspects, dealing with the requirements of the diagnostic methods, but also by the nature of the materials, the radio-opacity of the particles and the like. In general the amount is selected in such a way that it does not influence the visco-elastic behaviour of the gel too much. Between the broadest limits of the volume ratio of gel to particles of 1:100 and 100:1, the preferred ranges are between 20:1 to 1:1, more preferred between 9:1 and 3:1. When those ranges have been met, the core filling material of the invention will have all advantages discussed above, such as visco-elastic behaviour, radiopacity and the like.

In a further preferred embodiment, the radiopaque particles comprise at least one polymer, copolymer, terpolymer, or another synthetic material, which polymer, copolymer, terpolymer, or another synthetic material may be crosslinked, that owes the radiopacity to the presence of iodine as a part of the polymer structure, such that the iodine content of the particles is within the range 1- 50 % by mass, but preferably in the range 10 - 25 % by mass.

Further it is preferred that the radiopaque particles consist of a material that features both radiopacity (capacity to absorb X-radiation) and zero or negligible magnetic properties, such that no artefacts will occur during magnetic resonance imaging.

The radiopaque material discussed above may further be mixed with a radiopaque filling agent, such as, but not limited to- bariumsulfate, bariumoxide, zirconium dioxide, bismuth salts, iodine salts, organic iodine-containing molecules, gold, platinum, iridium or any other metal.

All components of the material of the invention have to be biocompatible. This is inherent to the specific use thereof. Further it should preferably be visco-elastic, i.e. it should have at least some shape retention properties.

A viscoelastic material is a material that exhibits both viscous and elastic characteristics when it is deformed through an external force. Viscous materials (like honey) deform linearly with time when a stress is applied. Elastic materials, on the other hand, deform instantaneously when stretched and they return quickly to their original state when the force is removed. Viscoelastic materials have elements of both of these properties, and (as such) exhibit time-dependent strain.

It should be stressed that, at best, the application of viscoelastic filling materials in breast prostheses at best help to approach the physical-mechanical properties of the natural breast tissue. It is well-known that the breast (and other soft tissues as well) exhibit so-called high-order mechanical properties, comprising non-linear elasticity and complex viscoelastic hysteresis. See, for instance: A. Gefen and B. Dilmoney. "Mechanics of the normal woman's breast" Technology in Health Care 15(4) pp 259-271 (2007).

Suitable materials for the gel part of the material of the invention are synthetic, natural or nature-derived material with optimised physical and mechanical properties in order to mimic the structure and softness of human breast tissue. Suitable examples are synthetic silicone rubber optionally having shape memory properties.

A preferred embodiment of this invention involves filling materials which are vulcanised in situ during the industrial production process. Examples of such filling materials are silicone rubbers. These materials are introduced into the capsule of the breast prosthesis as prepolymers. During the vulcanisation phase, crosslinks are generated, resulting in a three-dimensional macromolecular network with visco-elastic soft rubbery consistency. This has been described in the patent application WO/2008/052650: "Method for the production of a breast prosthesis" (publication dat 08.05.2008) and in the scientific references and prior art described therein. The radiopaque particles of this invention are introduced into the capsule of the breast prosthesis along with the prepolymer and it is ensured that the particles are distributed homogeneously. During the vulcanisation process, the radiopaque particles may or may not become attached to the filling material. In any case, the viscosity of the filling material which markedly increases as a consequence of the vulcanisation process ensures that the particles will remain evenly distributed over the volume of the breast prosthesis.

This invention accordingly deals a novel method for the imaging of (potentially) ruptured breast (and other) prostheses, through the use of computed tomography based on absorption of low-energy X-radiation. The method can be used as a stand-alone method of inspection of breast prostheses, or as an adjunct to MR imaging it its different modalities. A potential drawback of the method is the use of ionising X-radiation, although the energy of the radiation that is to be used can be kept very low. It should be remembered that inspection of breast tissues using low-energy X-radiation is common, e.g. in the screening for mamma carcinomas.

This invention is particularly relevant for silicone-filled breast prostheses, i.e., both silicone gel prostheses and cohesive gel prostheses. The principle of the invention is that radiopaque particles are introduced within the silicone gel material in such a way that the particles are homogeneously spread over the gel volume. A three-dimensional X-ray image of a breast that holds an implanted prosthesis according to the present invention will show the radiopaque particles, and therefore the volume of the inner gel, provided that the radiopaque particles are evenly distributed throughout the gel volume. Upon extracapsular leakage, both silicone gel and radiopaque particles will leak away from the site of implantation, and this will be directly visible on the three-dimensional image. Thus, inspection of the three-dimensional X-ray image of breast augmentation implants according to this invention provides direct information of the integrity of the prosthesis, AND -in the case of extracapsular leakage- information about the size and location of the amount of gel that has escaped from the capsule. This type of information is crucial in cases in which surgical removal of the ruptured prosthesis and silicone gel that has escaped from its original location is inevitable.

The viscosity of the silicone gel or the cohesive gel ensures that the radiopaque particles will remain homogenously spread over the inner volume of the breast prosthesis. Due to the relatively high viscosity of the silicone gel or the cohesive gel that forms the core of the breast prosthesis, there is only limited movement of the radiopaque particles relatively to each other possible. For instance, the radiopaque particles will not move to the lower part of the breast prosthesis because of the gravity force, and as a result of a possible difference in the specific density of gel material on one hand, and the radiopaque material on the other hand. There is no mechanism operative that can lead to coagulation of the radiopaque particles. Such coagulation would obviously disqualify the imaging method that of this invention.

The radiopaque particles must fulfil several requirements: (i) they must have sufficient X-ray contrast for fast and accurate imaging through the use of low-energy X-radiation; (ii) they should not dissolve in the silicone gel; (iii) they should be chemically inert; (iv) they should be biocompatible, which means that -in the case of extracapsular leakage- they should not cause any unwanted reaction in the tissues that surround the ruptured breast implant. Examples of such unwanted reactions are (but are not limited to): granuloma, inflammation, necrosis, apoptosis or formation of malignant tissue.

The invention is further directed to prostheses for various applications, including breast implants, other aesthetic, or reconstructive surgery. In general all these prostheses consist of an enveloping film, in which the core filling material is present. Examples thereof are augmentation of soft tissues or hard tissues such as bone, e.g. to replace damaged or diseased tissues, or to fill cavities after surgical excision of diseased tissues or malignant tissues, such as six-pack prostheses, chin prostheses, buttock prostheses, custom made prostheses for various defects, mostly post-traumatic or post oncologic.

Further, the following is remarked. In the event of a rupture of the wall of a breast prosthesis according to this invention, leading to extracapsular leakage, both silicone gel and radiopaque particles will reside in neighboring tissue. This is then detectable through computed tomography based on X-ray absorption. This is a routine clinical imaging technique, used frequently for the inspection of breast tissue. The presence of the silicone gel outside the prosthesis capsule is known be hazardous, as it can lead to granuloma and other complications. The presence of the radiopaque particles does not enhance the hazardous effect. In animal models, implantation of these radiopaque particles per se, or in a suspension of collagen, has consistently shown acceptance without any incompatibility effects.

Silicone gel or cohesive gel breast prostheses are manufactured in several steps. The penultimate step is the filling of the capsule with a silicone prepolymer and closure of the capsule. Then, a heat treatment is given (e.g. heating up to 80°C for 48 h). During this heat treatment, the prepolymer reacts further, leading to gellation of the core of the prosthesis. The core material then attains its desired physical properties, such as softness, shape memory, and natural feel). It is advisable to include the radiopaque polymeric particles already before loading of the capsule, i.e. also before the temperature-driven final polymerization of the silicone prepolymer. Special care should be given to prevent coagulation of the contrast particles during the gel formation, for instance through continuous rotation during the final heating phase. The use of the radiopaque particles of this invention is not expected to interfere with most methods to manufacture breast prostheses; see for instance: US patent 6,623,588 by L.B. Rasmussen, "Method of manufacturing a foil-wrapped breast prosthesis and a shaping tool for use in performing the method" (1998), and US patent 6,296,800 by N. Stelter et al. "Method for the manufacture of breast prostheses" (2000).
In the figures,
Figure 1 gives a schematic drawing (side-view) of the female breast with a synthetic implant for breast augmentation;
Figure 2 shows an example of a structural formula of a suitable iodine-containing methacrylate-type monomer which is suitable to prepare radiopaque microspheres for use in this invention;
Figure 3 is a photomicrograph: histological analysis of radiopaque microspheres after injection in subcutaneous tissue in a mouse. Follow-up time: 3 months. Note the absence of (i), any inflammatory response; (ii) granuloma; (iii), necrosis, in the area designated as "injected microspheres" Moreover, no unwanted responses were encountered in the neighboring tissues or more distant tissues either;

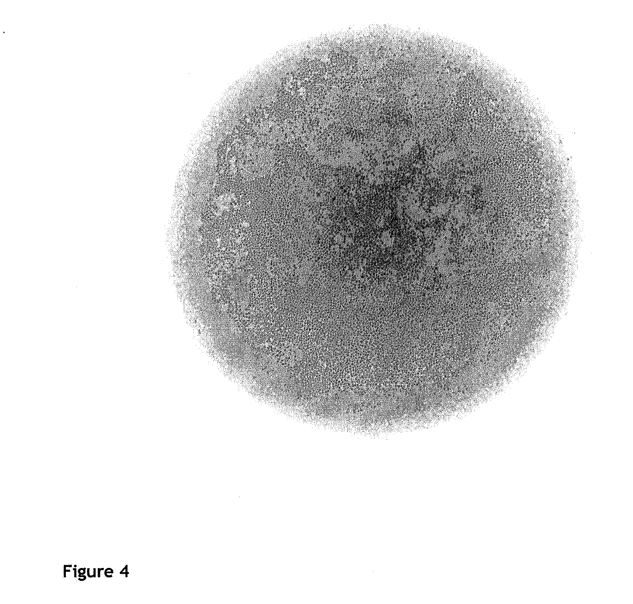

Figure 4 gives an X-ray image of the entire silicone-filled breast prosthesis. The core of the prosthesis contains silicone gel (approximately 95 % by volume) as well as radiopaque polymeric iodine-containing microspheres (approximately 5 % of the volume). The particles have a diameter in the range 600 - 800 microns. Note the clear visibility of the particles, while the breast prosthesis by itself is practically radiolucent, i.e. transparent for X-radiation). Note the clear visibility of the radiopaque polymeric microspheres. The image in Figure 4 provides a two-dimensional image of the breast prosthesis. The implication of this image (and analogous images) is that, upon using X-ray computed tomography (CT), it is possible to spatial information of the mixture of radiopaque microspheres and silicone gel in three dimensions. This can be used to obtain unequivocal information on the presence or absence of extracapsular leakage of breast prostheses after their implantation;
Figure 5 shows a detailed (ex vivo) X-ray images compared to Figure 4 and
Figure 6 is a photograph of the silicone-gel filled breast prosthesis, used to generate the X-ray images shown in Figure 4. Note that the detailed image was taken at the cross of the red lines, i.e. in the centre of the prosthesis.

### EXAMPLES

### Example 1. Use of polymer microspheres containing covalently bound iodine in their structure.

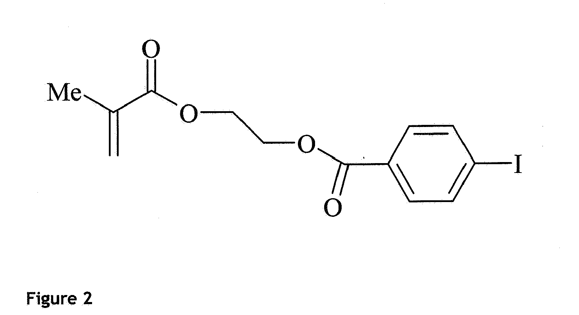

Especially useful for application in the context of this invention are polymeric microspheres as described in WO2008054205 ("Homogeneous intrinsic radiopaque embolic particles"; LH Koole and CSJ van Hooy-Corstjens); and in the scientific publications Biomacromolecules Vol 9, pp 84-90 (2008); Biomaterials Vol 28, pp 2457-64 (2007); Biomacromolecules Vol 7, pp 2991-6 (2006); Journal of Biomedical Materials Research Vol 73 pp 430-6 (2005). The polymeric microspheres, described in these works, exhibit clear X-ray contrast, due to the presence of iodine in their macromolecular structure. For example, an essential building block of these materials is the reactive methacrylate monomer 1 shown in Figure 2. Monomer 1 was usually reacted with another monomer, such as methylmethacrylate, 2-hydroxyethylmethacrylate or N-vinylpyrrolidinone. Typically, the content of iodine in the microspheres that were produced was in the range 1 - 30 percent by mass.

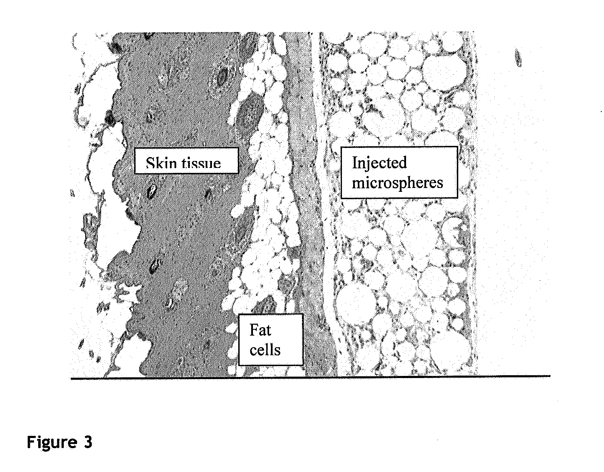

Furthermore, these microspheres were found to be non-toxic for contacting cells of various phenotypes in vitro. Consistently, after implantation in animals, there was complication-free acceptance of the particles in the surrounding tissues (both after subcutaneous implantation and upon intramuscular implantation). These observations were made after detailed histological examinations, and after follow-up times of three months after implantation. Figure 3 shows a detailed image of the acceptance of injected radiopaque polymeric microspheres in a mouse model (subcutaneous implantation, follow-up time 3 months).

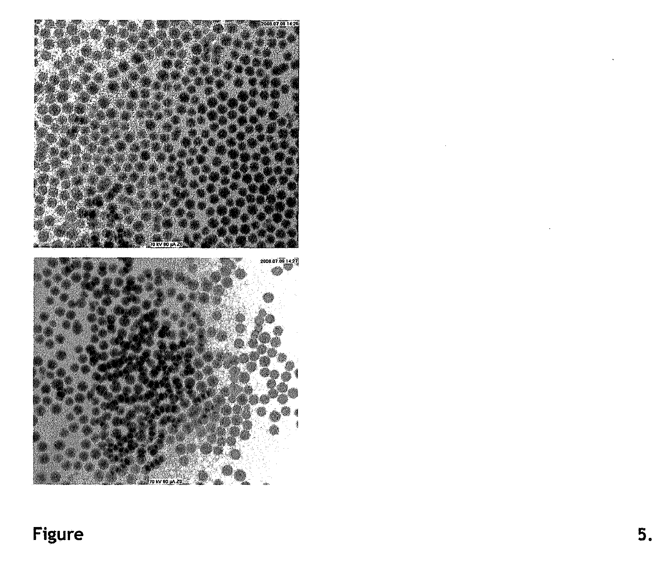

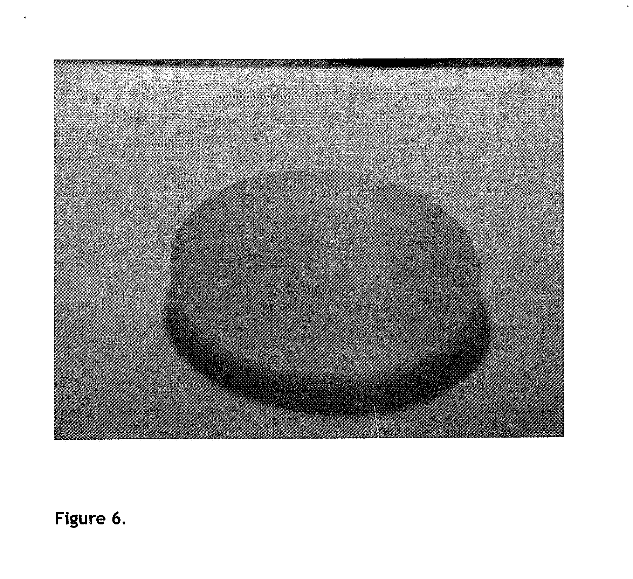

Figure 4 shows an X-ray image of a commercial breast prosthesis with silicone gel core; the silicone gel contains iodine-containing radiopaque polymeric particles, to a content of approximately 5 % by volume. The iodine content in the particles is 15 % by mass. The particles are distributed evenly over the volume of the silicone gel. Their diameter is in the range 800-1000 micrometers. Figure 5 is a more detailed image, as compared to Figure 4. The presence of the radiopaque particles left the softness and the pliability of the prosthesis completely unaltered. Exactly the same observations were made with breast prostheses of the cohesive gel type. Note that the images in Figures 4 and 5 are not generated through computed tomography. Both are two-dimensional X-ray pictures, which essentially show the shadows that are generated by the radiopaque particles. Figure 6 shows a photograpgh of the breast prosthesis, filled with a mixture of silicone gel and radiopaque particles that was used to generate Figures 4 and 5.

One further remark should be made. The radiopaque polymeric particles as described in this example do not possess magnetic properties. This implies that their presence in the breast prosthesis does not invoke any artifacts in images, produced through magnetic resonance imaging. This is a unique and important feature, since the use of other radiopaque particulate materials, notably metallic particles, will lead to such artefacts. The radiopaque polymeric particles as described in Example 1 combine the desired capability to efficiently absorb X-radiation, with the feature that these particles can also be visualized and/or localized visible through magnetic resonance imaging. Provided that the spatial resolution of the magnetic resonance imaging equipment is sufficiently high, the radiopaque polymer particles described in Example 1 are discernable as areas that are devoid of water. Their non-magnetic nature ensures that no artefacts (which could obscure other information in the images) can occur.

### Example 2. Use of polymer microspheres containing barium sulfate in their structure.

In this example, polymer microspheres that contain barium sulfate are used as the contrast particles. Like in example 1, the particles are to be added to the gel interior of breast prostheses. The most important advantages of the use of barium sulfate as the contrast agent is that it is readily available, cheap, and already widely used to introduce radiopacity into polymeric implant biomaterials. For example, bariumsulfate-filled poly(methyl methacrylate) is applied extensively as bone cements in orthopedic surgery. An attractive route to polymer particles, filled with barium sulfate, is the use of suspension polymerization. Then, a mixture of monomer (e.g., methyl methacrylate), a free-radical initiator (e.g., dibenzoylperoxide), a detergent, and finely dispersed bariumsulfate particles, is added dropwise to a stirred aqueous medium. Then, droplets containing bariumsulfate are formed. The droplets are stabilized by the detergent molecules, assembling at the monomer-water interface. Upon rising the temperature, polymerization occurs in each of the droplets. Usually, a reaction time of several hours is required to maximize the conversion. After cooling, the barium-sulfate containing particles are readily obtained, after washing and lyophilization. It should be noted that this method can be followed as well with other contrast agents, besides bariumsulfate. Other heavy element salts or oxides, such as bariumoxide, zirconiumdioxide, or bismuth salts, can also be used for this purpose. Alternatively, metallic particles with dimensions in the micrometer range and/or with dimensions in the nanometer range can be used for this purpose.

## Claims

1. Core filling material for prostheses, such as breast implants and implants for aesthetic and reconstructive surgery, comprising at least one biocompatible gel material and evenly dispersed through the gel material at least one particulate radiopaque material, wherein the core filling material is viscoelastic at 37°C, having a viscosity in the range of 10 to 10⁸ cP.

2. Core filling material according to claim 1, wherein the amount of radiopaque material is sufficient to provide spatial information about the presence of the gel material in non-invasive X-ray imaging of the radiopaque particles.

3. Core filling material according to claim 1 or 2, wherein the ratio of gel material: radiopaque particles (volume : volume) is in the range 100 : 1 to 1 : 100, preferably in the range 20 : 1 to 1 : 1, more preferred 9 : 1 to 3 : 1.

4. Core filling material according to claim 1-3, wherein the gel is a synthetic silicone rubber optionally having shape memory properties.

5. Core filling material according to claim 1-4, wherein the radiopaque particles are spheres with a diameter between 0.1 micrometer and 10 millimeter, preferably in the diameter range 50 micrometer - 1 millimeter.

6. Core filling material according to claim 1-5, wherein the radiopaque particles consist of a polymer, copolymer, terpolymer, or another synthetic material, which polymer, copolymer, terpolymer, or another synthetic material may be crosslinked, that owes the radiopacity to the presence of iodine as a part of the polymer structure, such that the iodine content of the particles is within the range 1 - 50 % by mass, but preferably in the range 10 - 25 % by mass.

7. Core filling material according to claim 1 - 6, in which the radiopaque particles consist of a material that features both radiopacity (capacity to absorb X-radiation) and zero or negligible magnetic properties, such that no artifacts will occur during magnetic resonance imaging.

8. Core filling material according to claiml-7, wherein the said radiopaque particles comprise a polymer, copolymer or terpolymer (crosslinked or uncrosslinked), mixed with a radiopaque filling agent, such as, but not limited to- bariumsulfate, bariumoxide, zirconium dioxide, bismuth salts, iodine salts, organic iodine-containing molecules, gold, platinum, iridium or any other metal.

9. Breast implant or prostheses, comprising at least one core filling material as defined in claims 1-8, inside an enveloping material.

10. Use of the core filling material of claim 1-8 in cosmetic or plastic surgery, e.g. for augmentation of soft tissues or hard tissues such as bone.

11. Use of the core filling material of claim 1-8 in corrective surgery, e.g. to replace damaged or diseased tissues, or to fill cavities after surgical excision of diseased tissues or malignant tissues, such as six-pack prostheses, chin prostheses, buttock prostheses, custom made prostheses for various defects, mostly post-traumatic or post oncologic.

12. Use of the core filling material of claim 1-8 in veterinary medicine.

13. Method for detecting the position, integrity or both of a breast implant or prostheses in the body of a mammal by X-ray diffraction, wherein the implant or prostheses is as defined in claim 8.
